# EUROPEAN PATENT APPLICATION

(11) **EP 0 752 252 A2**
(43) Date of publication of application: **08.01.1997**
(21) Application number: 96304196.7
(22) Date of filing: 06.06.1996
(51) Int. Cl.: A61M 25/06, A61M 5/32

(54) **Needle tip protector**

(30) Priority: 07.06.1995 US 482594
(71) Applicant: JOHNSON & JOHNSON MEDICAL, INC., Arlington, Texas 76004-3130 (US)
(72) Inventor: Chang, Joseph J., Avon, CT 06001 (US)
(74) Representative: Mercer, Christopher Paul

(57) **Abstract**

A needle tip protector (16) which assures that a needle (14) is secured and protected against accidental puncture after it is withdrawn from a patient's body. The needle tip protector includes a needle, having a needle tip (18) for initiating a body puncture, with a wire (20) attached to the needle at its end (22) opposite to the needle tip. A needle tip protector tubing (24) which is curved along its length surrounds the needle and has a fitting such as a Luer (26) fitting at a first end with a first through hole (28) having a diameter sufficiently large to enable the needle to pass therethrough. A cap (30) is provided at the second end of the protector tubing, with a second through hole (32) having a diameter sufficiently large to enable the wire to pass therethrough but small enough to prevent the needle from passing therethrough. The needle tip protector tubing has a length larger than the length of the needle, such that after a body puncture, the needle is withdrawn by pulling the wire and needle until the needle is stopped by the cap, at which position the entire needle is encased by the needle tip protector tubing, with the needle tip deflected to one side of the first through hole because of the curvature of the curved needle tip protector tubing. The needle tip protector can be used with a catheter, such as an intravenous (IV) or winged IV catheter, or with an endoscopic device.

## Description

### BACKGROUND OF THE INVENTION

### 1. Field of the Invention

The present invention relates generally to a needle tip protector, and more particularly pertains to a needle tip protector for use with a catheter, such as an intravenous (IV) or winged IV catheter, or with an endoscopic device, and assures that the needle is secured and protected against accidental puncture after it is withdrawn from the patient's body.

### 2. Discussion of the Prior Art

The present invention relates to clinical apparatus of the type in which pointed needles are used to puncture the skin of a patient, and in particular to catheters employing such needles to effectuate venous punctures.

It is well known and common practice by physicians to inject fluids and drugs directly into the bloodstream of patients. Also, during surgical dperations, it is frequently necessary to administer whole blood transfusions and parenteral fluids. Historically, introduction of such fluids into the cardiovascular system of a patient has required the making of a venipuncture using a hollow rigid needle having a proximal attachment site for fluid connecting the needle to a source of intravenous fluid or the like. This method of administering fluids created some persisting problems in the art. Primarily, the rigidity of the needle within the vein requires that the needle, usually on the arm, be maintained, for reasons of safety, in a fixed position at the general site of the venipuncture throughout the duration of fluid administration or transfusion, which may consume considerable time. Secondly, where it has been necessary to periodically draw blood samples and/or successively administer intravenous fluids, the patients may be required to experience a venipuncture each time, which repeated venipunctures are generally highly traumatic.

More recently it has been the practice to insert a flexible catheter tube into a vein and leave the catheter tube in such a position for purposes such as periodically administering fluids, transfusions and medication, collecting of blood samples, etc. In this way, the trauma, extravasation, infiltration, etc., of repeated venipunctures are avoided and the danger and discomfort of leaving a rigid needle in the body for a prolonged period of time are overcome. To place the distal end of such a flexible catheter tube within a body cavity, such as a vascular cavity, a cannulated or hollow needle is used to make the venipuncture. Thereafter following the venipuncture, the catheter tube, which is telescopically mounted with respect to the needle, is displaced relative to the needle into the vein of the patient. The needle may thereafter be completely removed from the catheter tube and disposed of. Having been in the patient's body, where it may have been exposed to infectious agents, the needle represents an infection hazard to clinical personnel if they should accidentally jab themselves with it after withdrawal.

Intravenous catheters for the infusion of fluids into the peripheral vein of a patient are frequently produced in two general forms: through-the-needle catheters, in which a catheter is threaded through the needle cannula and into the vein of a patient, and over-the-needle catheters, in which the needle and a concentric outer catheter are inserted into the vein and the needle is then withdrawn from the emplaced catheter.

A typical over-the-needle IV catheter requires the user to remove and then dispose of a contaminated needle after the needle tip and catheter are properly located in the vein of the patient. Once the needle is withdrawn from the catheter, the user's immediate priorities are infusion set connection and site preparation, including the taping of the catheter to the patient. Because of the urgency of these procedures, the needle is normally just dropped conveniently nearby and then retrieved later. Since the needle at this time is exposed and located close tow here the user is completing work with the catheter, accidental self-inflicted needle injuries are not uncommon.

The possibility that clinical personnel might contract conditions such as AIDS or hepatitis through accidental punctures by used needles has been regarded seriously. Accordingly, a significant body of prior art has been developed for preventing such accidental punctures.

Unfortunately, almost none of the prior art development succeeded in producing a device in which the withdrawal of the needle from the patient's body automatically activated a protective mechanism. In each case, it was necessary for the clinical personnel to consciously perform an extra step in order to invoke the protection offered by the prior art.

One example is U.S. Patent 4,631,057 to Mitchell which discloses a guard tube capable of sliding forward to protect the pointed end of a hypodermic needle from accidental contact after usage. This mechanism, however, like the rest of the prior art, is only effective if the clinical personnel remember to push the guard tube into its effective position after performing an injection. There is a strong possibility that they will occasionally forget to do this.

Now that the range of conditions to which clinical personnel are exposed as a result of accidental needle punctures includes the condition AIDS, it is even more important to provide a safety mechanism which offers such personnel fail-safe protection, i.e. a device which operates without the need for conscious forethought on their part, a mechanism which automatically protects the pointed end of a needle from the moment when it is withdrawn from the body of a patient.

McDonald U.S. Patent 4,944,725 also addresses this problem, and discloses an intravenous catheter which protects a clinician from accidental puncture which may result in the transfer of dangerous infections. The catheter is introduced with the aid of a needle, which is thereafter withdrawn from the patient's body into a protective housing without exposing the needle during any intermediate stage of the process. The housing is latched in place after needle withdrawal, and for unlocking a catheter hub in place after that time, and withdrawal and locking are effected in one continuous motion.

Winged IV set catheters have been on the market for several years. One brand familiar to the IV therapist is the Angio-set manufactured pursuant to U.S. Patent 4,177,809, issued December 11, 1979. This patent discloses an intravenous catheter comprising an intravenous catheter tube, and a manually guidable circumferentially deformable elastomeric bore-defining tubular inserter integral with and in axial alignment with the catheter tube. A needle is positioned concentrically within the catheter tube and inserter, and extends through at least part of the bore of the inserter to beyond the distal end of the catheter tube. The inserter comprises opposed laterally extending wings adapted to be manually compressed together from an at-rest position to a needle control position. In the at-rest position, the bore of the inserter is sufficiently large to permit retraction of the needle from the catheter tube and the inserter. When the inserter wings are compressed to the needle control position, the bore of the inserter is sufficiently constricted such that the inserter grips the needle firmly to permit inserter-forced injection of the distal end of the needle into the body of a patient. A device as disclosed therein can be easily modified to include a winged IV set needle tip protector pursuant to the teachings of the present invention.

### SUMMARY OF THE INVENTION

Accordingly, it is a primary object of the present invention to provide a needle tip protector which assures that the needle is secured and protected against accidental puncture after it is withdrawn from the patient's body.

The present invention can be used with a catheter, such as an intravenous (IV) or winged IV catheter, or with an endoscopic device. In a winged IV set catheter a needle having a pointed forward end is inserted therethrough and is adapted to puncture the skin of a patient. The winged IV set catheter is secured to the needle rearwardly of the pointed end, and is adapted for manually pushing the needle forwardly in order to effectuate an intentional puncture, and thereafter for manually pulling the needle rearwardly in order to withdraw it from the puncture site. A curved protective tubing is provided which has a curved tubular needle-receiving passage therein. The passage is sufficiently long to permit the entire length of the needle to be withdrawn rearwardly into the interior thereof to prevent subsequent accidental punctures by the encased needle tip.

In accordance with the teachings herein, the present invention provides a needle tip protector which comprises a needle, having a needle tip for initiating a body puncture, with a lead such as a wire attached to the needle at its end opposite to the needle tip. A needle tip protector tubing which is curved along its length surrounds the needle and has a fitting at a first end with a first through hole having a diameter sufficiently large to enable the needle to pass therethrough, and having a cap at its second end, opposite the first end, with a second through hole Shaving a diameter sufficiently large to enable the lead to pass therethrough but small enough to prevent the needle from passing therethrough. The needle tip protector tubing has a length larger than the length of the needle, whereby after a body puncture, the needle is withdrawn by pulling the lead and needle until the needle is stopped by the cap, at which position the entire needle is encased by the needle tip protector tubing, with the needle tip deflected to one side of the first through hole because of the curvature of the curved needle tip protector tubing.

In greater detail, the fitting comprises a Luer adaptor fitting. In a second embodiment, an inwardly extending duckbill fitting is positioned at the first through hole to further prevent the needle tip from re-entering the first through hole. Moreover, the needle tip protector can be used with an intravenous catheter such as a winged intravenous catheter set which includes a winged fitting comprising an elastic tubular body which is reversibly deformable to and from (a) an at-rest open position in slidable relation with the needle and (b) a laterally constricted position around the needle. In this arrangement, the fitting has opposed wings adapted to be gripped for manipulating the catheter set, with the wings being squeezable together to cause the fitting body to be deformed to the laterally constricted position around the needle such that the fitting is prevented from sliding relative to the needle.

Alternatively the needle tip protector can be used with an endoscopic device such as an endoscopic device having a soft point such as is used to penetrate a gall bladder.

### BRIEF DESCRIPTION OF THE DRAWINGS

The foregoing objects and advantages of the present invention for a needle tip protector may be more readily understood by one skilled in the art with reference being had to the following detailed description of several preferred embodiments thereof, taken in conjunction with the accompanying drawings wherein like elements are designated by identical reference numerals throughout the several views, and in which:
Figure 1 illustrates a needle stick protection feature pursuant to the teachings of the present invention in which as the needle is withdrawn during threading of a catheter the entire needle is encased in a needle protection system;
Figure 2 illustrates the needle protection system in a locked position; and
Figure 3 illustrates a further embodiment of the present invention similar to Figures 1 and 2 in which a duckbill-like rubber component is placed at the distal end of the adaptor through-hole to further prevent the needle tip from entering the through-hole.

### DETAILED DESCRIPTION OF THE DRAWINGS

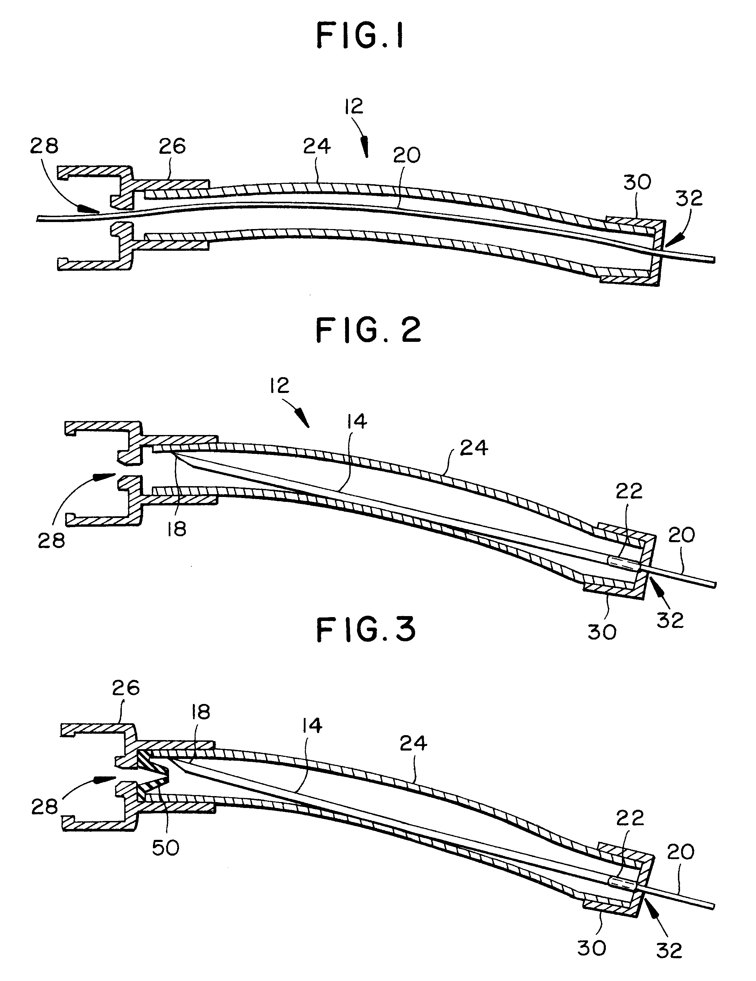

Referring to the drawings in detail, Figure 1 illustrates a device 12 having a needle stick protection feature pursuant to the teachings of the present invention. Figure 2 illustrates the device 12 after a needle 14 has been withdrawn from a catheter and the entire needle 14 is encased in a needle protection system.

The device 12 comprises a needle 14, having a needle tip 18 for initiating a body puncture, with a lead such as a wire 20 attached to the needle at its end 22 opposite to the needle tip. A needle tip protector tubing 24 which is curved along its length surrounds the needle and has a well known Luer fitting 26 at a first end with a first through hole 28 having a diameter sufficiently large to enable the needle 14 to pass therethrough, and having a cap 30 at its second end, opposite the first end, with a second through hole 32 having a diameter sufficiently large to enable the wire 20 to pass therethrough but small enough to prevent the needle 14 from passing therethrough. The needle tip protector tubing 24 has a length larger than the length of the needle, whereby after a body puncture, the needle 14 is withdrawn by pulling the wire and needle until the needle is stopped by the cap 30, at which position (Figure 4) the entire needle 14 is encased by the needle tip protector tubing 24, with the needle tip 18 deflected to one side of the first through hole 28 because of the curvature of the curved needle tip protector tubing.

The Luer attachment fitting 26 can have a slip or a lock fit to prevent blood from leaking out of a Y-connector, not illustrated. It does not require tight dimensional tolerances and can be made of an inexpensive materials such as Latex or K-resin. The semi-rigid tubing section 24 can be formed of Tygon^{TM} or other appropriate tubing stock. The cap 30 can be formed of a high modulus material such as acrylic or ABS.

In usage, a catheter device is inserted using the same technique as used with a current winged IV set. After venipuncture is verified and a desired insertion depth is achieved, the needle 14 can be withdrawn by pulling the needle/wire hub until the needle is caught by the cap 30. The entire needle is then encased in the needle protection system as shown in Figure 2. Because of the curved length of the semi-rigid tubing 24, the needle tip 18 is always deflected to one side. Unless an extra effort is made, it is unlikely for the needle to be pushed back through the Luer adapter 26 and become unprotected.

Figure 3 illustrates a further embodiment in which a simple device such as a duckbill-like rubber component 50 placed at the distal end of the adaptor through-hole. Once the needle tip 18 is withdrawn beyond the duckbill 50, it is virtually impossible for the needle tip to re-enter the duckbill 50 through the distal end and stick out the adaptor.

The needle tip protector of the present invention can be used with a catheter, such as an intravenous (IV) or winged IV catheter, or with an endoscopic device.

While several embodiments and variations of the present invention for a needle tip protector are described in detail herein, it should be apparent that the disclosure and teachings of the present invention will suggest many alternative designs to those skilled in the art.

## Claims

1. A needle tip protector comprising:
a. a needle, having a needle tip for initiating a body puncture, with a lead attached to the needle at the needle end opposite to the needle tip;
b. a needle tip protector tubing which is curved along its length surrounding the needle and having a fitting at a first end with a first through hole having a diameter sufficiently large to enable the needle to pass therethrough, and having a cap at a second end, opposite the first end, with a second through hole having a diameter sufficiently large to enable the lead to pass therethrough but small enough to prevent the needle from passing therethrough, and having a length larger than the length of the needle, wherein after a body puncture, the needle is withdrawn by pulling the lead and needle into the tubing until the needle is stopped by the cap, at which position the entire needle is encased by the needle tip protector tubing with the needle tip deflected to one side of the first through hole because of the curvature of the curved needle tip protector tubing.

2. A needle tip protector as claimed in claim 1, wherein the fitting comprises a Luer adaptor fitting.

3. A needle tip protector as claimed in claim 1, wherein an inwardly extending duckbill fitting is positioned at the first through hole to further prevent the needle tip from re-entering the first through hole.

4. A needle tip protector as claimed in claim 1 in combination with an intravenous catheter.

5. A needle tip protector as claimed in claim 4, wherein the catheter is a winged intravenous catheter which includes a winged fitting comprising an elastic tubular body which is reversibly deformable to and from (a) an at-rest open position in slidable relation with the needle and (b) a laterally constricted position around the needle, wherein the fitting has opposed wings adapted to be gripped for manipulating the catheter set, the wings being squeezable together to cause the fitting body to be deformed to the laterally constricted position around the needle such that the fitting is prevented from sliding relative to the needle.

6. A needle tip protector as claimed in claim 1, in combination with an endoscopic device.
